# EUROPEAN PATENT APPLICATION

(11) **EP 4 257 688 A1**
(43) Date of publication of application: **11.10.2023**
(21) Application number: 21909136.0
(22) Date of filing: 07.12.2021
(51) Int. Cl.: C12N 15/63, A61P 31/18, C12N 5/10, G01N 33/569, A61K 39/42, C07K 16/10, C12Q 1/70, C12Q 1/68, C12N 7/01, C12N 15/13, C12R 1/93, C12N 1/21, C12N 1/19

(54) **NUCLEIC ACID CONSTRUCT FOR AIDS GENE THERAPY**

(30) Priority: 21.12.2020 CN 202011518828
(71) Applicant: Kanglin Biotech (Hangzhou) Co., Ltd., Hangzhou, Zhejiang 310018 (CN)
(72) Inventor: WU, Haoquan, Hangzhou Economic And Technological Development Zone Hangzhou, Zhejiang 310018 (CN); SUN, Baozhen, Hangzhou Economic And Technological Development Zone Hangzhou, Zhejiang 310018 (CN); DANG, Ying, Hangzhou Economic And Technological Development Zone Hangzhou, Zhejiang 310018 (CN)
(74) Representative: Canzler & Bergmeier Patentanwälte Partnerschaft mbB
(86) International application number: PCT/CN2021/136153
(87) International publication number: WO 2022/135139

(57) **Abstract**

Provided is a nucleic acid construct for AIDS gene therapy. The nucleic acid construct comprises multiple polynucleotides encoding single chain variable region fragments of an anti-AIDS neutralizing antibody, and polynucleotides encoding immunoglobulin Fc fragments; the single chain variable region fragments of the anti-AIDS neutralizing antibody comprise a single chain variable region fragment capable of specifically binding to HIV, and a single chain variable region fragment capable of specifically binding to a CD4 receptor. The construct can be used for gene therapy of AIDS caused by HIV infection, and the construct can be used for expressing an amphotropic/polytropic neutralizing antibody having broad-spectrum and high-efficiency neutralizing activity in vitro and in vivo and can be used for clinical research and new drug development of AIDS gene therapy drugs delivered by recombinant viral or non-viral vectors.

## Description

### TECHNICAL FIELD

The present disclosure relates to the technical field of biomedicine, in particular to a nucleic acid construct for AIDS gene therapy.

### BACKGROUND

Acquired immune deficiency syndrome (AIDS) caused by human immunodeficiency virus (HIV) infection is one of the biggest infectious diseases in the world today. With the development of anti-AIDS drugs, the expected lifetime of AIDS infectors who have been actively treated has been greatly extended. However, the side effects and limitations of the cocktail therapy as well as the emergence of drug-resistant strains still cause long-term economic and social burdens, obvious decline in quality of life and obvious pain to AIDS patients.

Broadly neutralizing antibodies (bNAb) with HIV-I neutralizing activity produced by a small number of elite infectors over several years can efficiently and broadly bind to HIV surface glycoproteins and neutralize the infective activity of HIV to CD4+T cells, which is a promising method to prevent or resist HIV-I infection. However, the specific mechanism of their production is not clear. Most HIV-I infectors can only produce non-neutralizing antibodies. At present, no study has induced the production of broad-spectrum neutralizing antibodies in healthy subjects by standard immunization methods.

A large number of clinical stage studies show that the recombinant broad-spectrum neutralizing antibodies targeting HIV-I virus can effectively reduce the virus load in patients. Even if they can not completely eliminate HIV in the body, they can help to control the development of AIDS. Compared with small molecular anti-AIDS virus drugs, the recombinant neutralizing antibodies can greatly reduce toxic and side effects, and increase the administration compliance of patients. Clinically, recombinant broad-spectrum neutralizing antibodies can be used not only as vaccine substitute products in specific cases to prevent AIDS, but also as antivirus drugs in different stages of diseases, thus becoming a hot spot in the development of anti-AIDS drugs.

At present, the only clinically approved anti-AIDS neutralizing antibody is Ibalizumab targeting CD4 receptors, which shows excellent therapeutic effect on AIDS patients with multiple drug resistance.

However, similar to high-efficiency anti-AIDS virus small molecule drugs, a specific broad-spectrum neutralizing antibody only targets a certain single epitope of a single virus protein (HIV-gp160), and the escape phenomenon caused by virus mutation is still inevitable. Moreover, because the development, production and use costs of broad-spectrum neutralizing antibodies are all much higher than those of small molecule anti-AIDS virus drugs, combined administration has no advantages. Therefore a large number of anti-AIDS broad-spectrum neutralizing antibody drugs under research are difficult to be used in clinics so far.

In light of the above problems, the development of anti-AIDS broad-spectrum neutralizing antibody drugs mainly needs to make breakthroughs in the following two aspects:

1. Development of bispecific and polyspecific neutralizing antibodies or antibody-like macromolecule drugs to cover the escape caused by virus mutation with multiple targets.

2. Genetic manipulation of protein broad-spectrum neutralizing antibody drugs. AIDS gene therapy drugs delivered by recombinant viral or non-viral vectors stably express neutralizing antibodies or antibody-like macromolecules in the body for a long time in a genomic integration or non-integration manner. One therapy is effective for a long time or even for life, greatly reducing the production and use costs of macromolecular antibody drugs.

### SUMMARY

In view of the shortcomings of the prior art mentioned above, the purpose of the present disclosure is to provide a nucleic acid construct for AIDS gene therapy, which is used to solve the problems in the prior art.

In order to achieve the above objects and other related objects, the present disclosure provides a nucleic acid construct. The nucleic acid construct includes multiple polynucleotides encoding single chain variable region fragments of an anti-AIDS neutralizing antibody, and a polynucleotide encoding an immunoglobulin Fc fragment; the single chain variable region fragments of the anti-AIDS neutralizing antibody include a single chain variable region fragment capable of specifically binding to HIV, and a single chain variable region fragment capable of specifically binding to a CD4 receptor.

The present disclosure also provides a lentivirus, which is formed by packaging the nucleic acid construct by a virus.

The present disclosure also provides a lentiviral vector system, which includes the nucleic acid construct and an auxiliary plasmid.

The present disclosure also provides an adeno-associated virus, which is formed by packaging the nucleic acid construct by a virus.

The present disclosure also provides an adeno-associated viral vector system, which includes the nucleic acid construct and an auxiliary plasmid.

The present disclosure also provides a bispecific antibody for preventing or treating AIDS, which is an antibody produced by infecting host cells with the lentivirus or adeno-associated virus; preferably, the amino acid sequence of the antibody is shown as SEQ ID NO: 5 or SEQ ID NO: 7.

The present disclosure also provides a composition for preventing or treating AIDS, the effective substances of which include the nucleic acid construct; and/or the lentivirus, and/or the adeno-associated virus.

The present disclosure also provides a cell line, which is a cell line infected by the lentivirus or the adeno-associated virus.

The present disclosure also provides the use of the nucleic acid construct, the lentivirus, the lentiviral vector system, the adeno-associated virus, the antibody or the cell line in preparing drugs for preventing and treating AIDS.

The present disclosure also provides a method for treating AIDS, which includes administering an effective amount of the bispecific antibody to a patient.

Single chain antibody variable region fragments (scfv) of a plurality of broad-spectrum neutralizing antibodies and of the neutralizing antibody Ibalizumab targeting a CD4 receptor, together with Fc fragments of a humanized antibody are combined into a simple and high-efficiency expression cassette in the present disclosure. The experiment results show that the expression efficiency of the expression cassette after being delivered by the recombinant viral vector is significantly higher than that of the traditional antibody expression cassette.

Among the various combinations of single chain antibody variable region fragments (scfv) of different broad-spectrum neutralizing antibodies and Ibalizumab, the combination of 10E8V4-5R+100cF and Ibalizumab shows much better in vitro cytological HIV-I virus neutralizing activity than other scfv combinations, and shows a broad-spectrum neutralizing ability against both CXCR4 and CCR5 viruses with two tropisms. The scfv combination bispecific antibody molecule based on the above expression cassette shows an effective blood drug concentration after being delivered into mice by lentivirus and adeno-associated viral vectors.

As mentioned above, the construct and the bispecific antibody of the present disclosure have the beneficial effect of developing a technical route for anti-AIDS broad-spectrum neutralizing antibody gene drugs of great potential. The application range of the present disclosure includes various AIDS gene therapies based on the gene expression of broad-spectrum neutralizing antibodies, including the gene therapy of AIDS caused by HIV infection. The construct can be used for expressing a bispecific/polyspecific neutralizing antibody having broad-spectrum and high-efficiency neutralizing activity in vitro and in vivo. It can be used for clinical research and new drug development of AIDS gene therapy drugs delivered by recombinant viral or non-viral vectors.

### BRIEF DESCRIPTION OF THE DRAWINGS

Fig. 1A shows the plasmid map of pKL-kan-lenti-EF1α-WPRE of the present disclosure.
Fig. 1B shows the anti-plasmid map of pAAV-MCS-CMV-EGFP of the present disclosure.
Fig. 2A shows the schematic diagram of the expected molecular structure of the bispecific anti-HIV neutralizing antibody-like molecule of the present disclosure.
Fig. 2B shows the schematic diagram of the expected molecular structure of the anti-HIV broad-spectrum neutralizing antibody of the present disclosure.
Fig. 3A shows the gene design for expressing the HIV neutralizing antibody KL-BsHIV01 of the present disclosure.
Fig. 3B shows the gene design for expressing the HIV neutralizing antibody 10E8V4-5R+100cF of the present disclosure.
Fig. 4A shows the adeno-associated virus gene therapy vector construct of the bispecific anti-HIV neutralizing antibody-like molecule KL-BsHIV01 of the present disclosure.
Fig. 4B shows the lentivirus gene therapy vector construct of the bispecific anti-HIV neutralizing antibody-like molecule KL-BsHIV01 of the present disclosure.
Fig. 4C shows the lentivirus gene therapy vector construction of the HIV neutralizing antibody 10E8V4-5R+100cF of the present disclosure.
Fig. 5 shows the non-reducing gel electrophoresis (Westernblotting) of the expression of the HIV neutralizing antibody by the gene therapy vector construct of the HIV neutralizing antibody of the present disclosure.
Fig. 6 shows the expression of the adeno-associated virus gene therapy vector construct of the HIV neutralizing antibody of the present disclosure in NSG mice.

### DETAILED DESCRIPTION

Unless otherwise defined below, all technical and scientific terms mentioned in the present disclosure have the meanings commonly understood by those skilled in the art.

The term "nucleic acid construct" refers to an artificially constructed nucleic acid segment that can be introduced into a target cell or tissue. The nucleic acid construct can be a lentiviral vector including a vector framework, i.e., an empty vector, and an expression cassette.

The term "vector" refers to a nucleic acid fragment or polynucleotide fragment used to introduce or transfer one or more nucleic acids or one or more polynucleotides into a target cell or tissue. Typically, a vector is used to introduce exogenous DNA into another cell or tissue. A vector can contain a bacterial resistance gene for growing in bacteria and a promoter for expressing a target protein in an organism. DNA can be produced in vitro by PCR or any other suitable technique(s) known to those skilled in the art.

The term "expression cassette" refers to a sequence that has the potential to encode a protein.

The present disclosure firstly provides a nucleic acid construct. The nucleic acid construct includes multiple polynucleotides encoding single chain variable region fragments of an anti-AIDS neutralizing antibody, and a polynucleotide encoding immunoglobulin Fc fragments; the single chain variable region fragments of the anti-AIDS neutralizing antibody include a single chain variable region fragment capable of specifically binding to HIV, and a single chain variable region fragment capable of specifically binding to a CD4 receptor.

In one embodiment, the single chain variable region fragment capable of specifically binding to HIV is selected from the single chain variable region fragments capable of specifically binding to AIDS virus envelope proteins.

Further, the single chain variable region fragment capable of specifically binding to HIV is selected from the single chain variable region fragments of antibodies capable of specifically binding to one or more of the following regions: the V1V2 glycan region, the V3 glycan region, gp160, gp120, the junction site of gp120 and gp41, or the membrane proximal external region on gp41.

In one embodiment, the single chain variable region fragment of the antibody capable of specifically binding to the V1V2 glycan region (V1V2-glycan) is selected from PG9, PG16, PGT141-145, CH01-04, and PGDM1400.

In one embodiment, the single chain variable region fragment of the antibody capable of specifically binding to the V3 glycan region is selected from PGT128, PGT121-123, PGT125-131, PGT135, 10-1074, and 2G12.

In one embodiment, the single chain variable region fragment of the antibody capable of specifically binding to the membrane proximal external region (MPER) on gp41 is selected from the single chain variable region fragments of 10E8V4-5R+100cF, 4E10, and 2F5.

In one embodiment, the single chain variable region fragment capable of specifically binding to the CD4 receptor is selected from the single chain variable region fragments of ibalizumab. Ibalizumab can interfere with the binding of the HIV surface glycoprotein to the CD4 receptor by binding to the CD4 receptor, thus neutralizing the infectivity of the virus to CD4+T cells.

The nucleic acid construct is a non-viral vector or a viral vector.

In a preferred embodiment, the viral vector is a lentiviral vector or an adeno-associated viral vector. Each polynucleotide is located in the expression cassette of the lentiviral vector or the adeno-associated viral vector.

In one embodiment, the nucleotide sequence of the nucleic acid construct is shown as SEQ ID NO: 6 or SEQ ID NO: 8.

The HIV refers to the human immunodeficiency virus, i.e., the AIDS virus, which is divided into type 1 and type 2.

The nucleic acid construct is a nucleic acid construct for AIDS gene therapy.

The present disclosure also provides a lentivirus, which is formed by packaging the nucleic acid construct by a virus.

The present disclosure also provides a lentiviral vector system, which includes the nucleic acid construct and an auxiliary plasmid.

Further, the auxiliary plasmid encodes one or more nucleotide sequences of gag and pol proteins as well as other necessary nucleotide sequences of virus packaging components. The auxiliary plasmid may include packaging plasmids and envelope plasmids.

The present disclosure also provides an adeno-associated virus, which is formed by packaging the nucleic acid construct by a virus.

The present disclosure also provides an adeno-associated viral vector system, which includes the nucleic acid construct and an auxiliary plasmid.

Further, the lentiviral vector system also includes a host cell, which can be selected from various applicable host cells in the field, as long as it does not limit the purpose of the present disclosure. Specific applicable cells can be lentivirus-producing cells, such as 293T cells.

The present disclosure also provides a bispecific antibody for preventing or treating AIDS, which is an antibody produced by infecting host cells with the lentivirus or adeno-associated virus.

The bispecific antibody is different from the traditional antibody, but a kind of antibody similar to the traditional antibody, so it can also be called as a bispecific anti-HIV neutralizing antibody. The structure of the bispecific antibody is that the antigen binding region consists of single chain variable region fragments (scfv) of two monoclonal broad-spectrum neutralizing antibodies linked in series by a linker, and then the antigen binding region is linked to the Fc fragment of the constant region of a humanized IgG antibody by a linker. This structure is beneficial to the formation of bispecific antibody molecule dimers and increases the plasma half-life of macromolecular drugs.

The linker peptide generally consists of amino acids with small side chains. For example: (Gly)4, GSGGSG, GSGGSGG GSGGSGGG, GGGGSGGG, and (GGGGS)3.

In one embodiment, the amino acid sequence of the bispecific antibody is shown as SEQ ID NO: 5 or SEQ ID NO: 7.

The present disclosure also provides a composition for preventing or treating AIDS, the effective substances of which contain the nucleic acid construct; and/or the lentivirus, and/or the adeno-associated virus.

The composition can be a pharmaceutical composition.

The form of the composition is not particularly limited, and can be various substance forms such as solid, liquid, gel, semi-fluid, aerosol and so on.

When the composition is used for preventing or treating AIDS, it is necessary to administer an effective dose of the composition to a subject.

The present disclosure also provides a cell line, which is a cell line infected by the lentivirus or the adeno-associated virus.

The present disclosure also provides the use of the nucleic acid construct, the lentivirus, the lentiviral vector system, the adeno-associated virus, the antibody or the cell line in preparing drugs for preventing and/or treating AIDS.

The present disclosure also provides a therapy method for AIDS, which includes administering an effective amount of the bispecific antibody to a patient.

The detailed description includes constructing a series of constructs of monospecific/bispecific antibodies and antibody molecules, and cloning them into recombinant adeno-associated virus vectors and recombinant lentiviral vectors; packaging the corresponding adeno-associated virus vector and lentiviral vector in 293T cells, and infecting the 293T cells as well as differentiated and undifferentiated muscle cell lines with a certain biological titer of viruses; and detecting quantitatively and qualitatively the produced antibody molecules in the cell supernatant, and testing their neutralizing activity against the HIV-1 wild virus in the analysis of infection activity of the HIV-1 wild virus strain and the virus-sensitive reporter gene cell line TzmbL.

The embodiments of the present disclosure are illustrated below through specific examples, and those skilled in the art can easily understand other advantages and effects of the present disclosure from the contents disclosed in this specification. The present disclosure can also be implemented or applied through other different detailed description, and various details in this specification can be modified or changed based on different viewpoints and applications without departing from the spirit of the present disclosure.

Before further describing the detailed description of the present disclosure, it will be understood that the protection scope of the present disclosure is not limited to the following specific embodiments; also, it will be understood that the terms used in the examples of the present disclosure is for the purpose of describing specific embodiments, and not for the purpose of limiting the protection scope of the present disclosure; and in the specification and claims of the present disclosure, the singular forms "a", "an" and "this" include the plural form unless the context clearly indicates otherwise.

When the examples give numerical ranges, it will be understood that unless otherwise specified in the present disclosure, two endpoints of each numerical range and any numerical value between the two endpoints can be selected. Unless otherwise defined, all technical and scientific terms used in the present disclosure have the same meaning as commonly understood by those skilled in the art. In addition to the specific methods, equipment and materials used in the examples, according to the mastery of the prior art by those skilled in the art and the records of the present disclosure, any methods, equipment and materials in the prior art similar to or equivalent to the methods, equipment and materials described in the examples of the present disclosure can be used to achieve the present disclosure.

### Example 1: Structural design for anti-HIV neutralizing antibodies and bispecific anti-HIV neutralizing antibody-like molecules

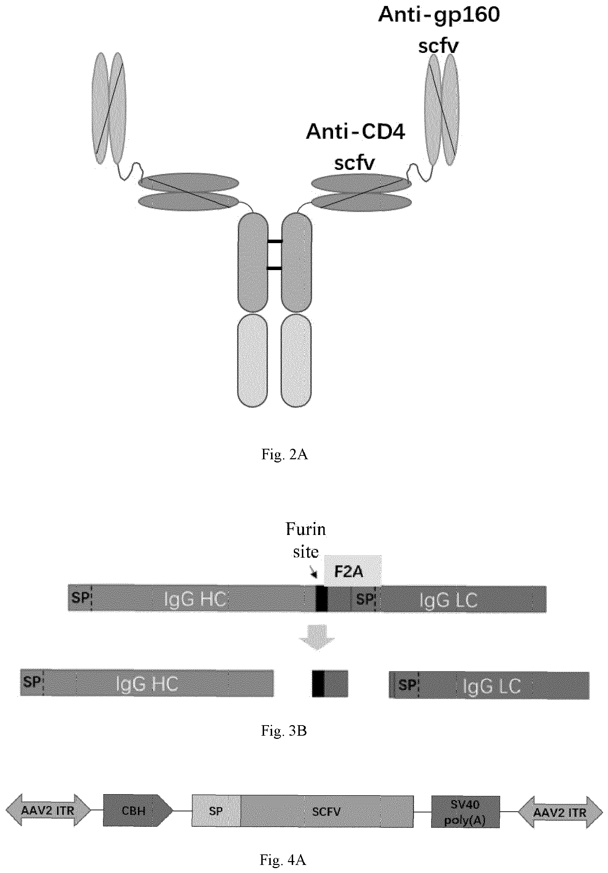

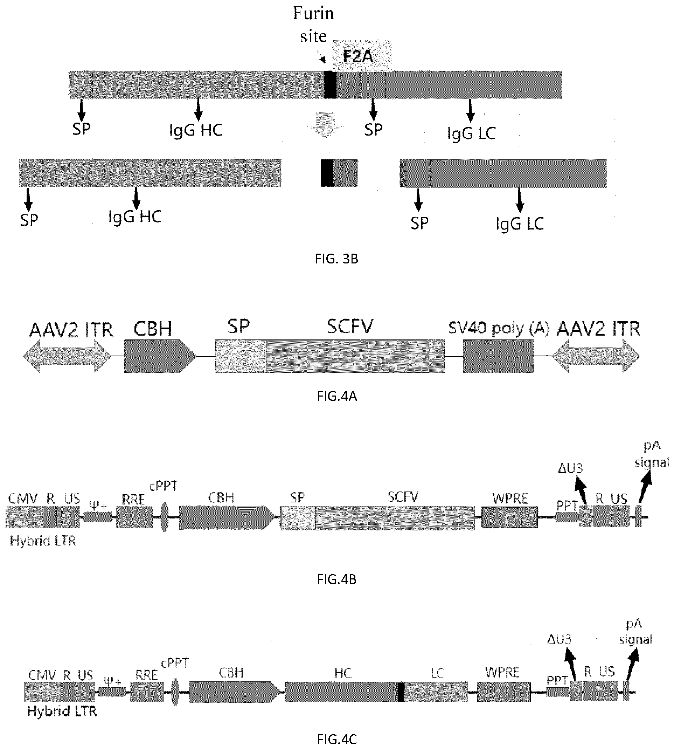

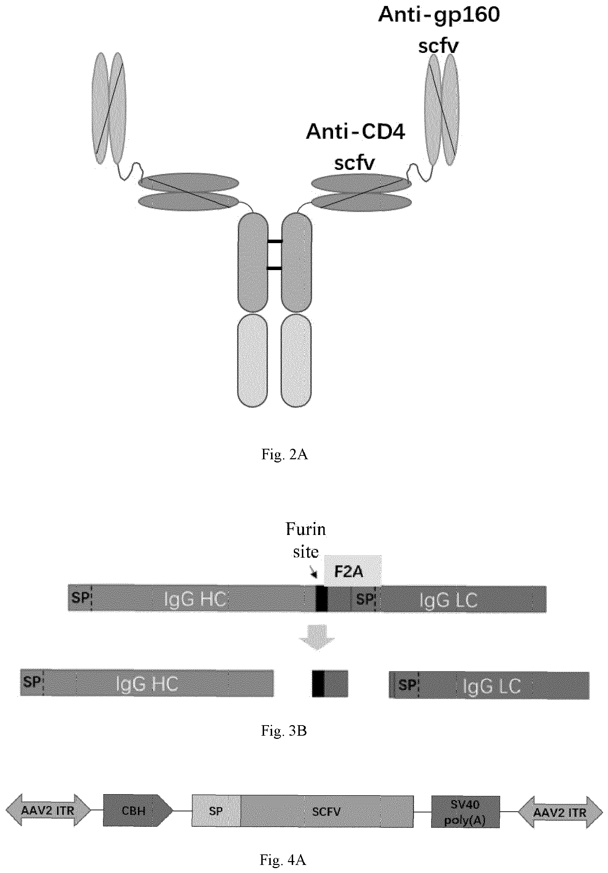

The structures of the anti-HIV neutralizing antibody and the bispecific anti-HIV neutralizing antibody-like molecule applied in the present disclosure are as follows:
1. The bispecific anti-HIV neutralizing antibody-like molecule: from the N terminal to the C terminal of the protein molecule, respectively: signal peptide-single chain variable region fragment of anti-HIV-gp160 broad-spectrum neutralizing antibody (VH-ggggsnlinker-VL)-ggggsnlinker-single chain variable region fragment of anti-human CD4 antibody (VH-ggggsnlinker-VL)-ggggsnlinker-human IgG1 CH2-human IgG1 CH3. The sequence shown was translated as a dimer in the cell and secreted from the cell. The structure of the expected mature molecule is shown in Fig. 2A, and the gene structure is shown in Fig. 3A.
2. The anti-HIV broad-spectrum neutralizing antibody: from the N terminal to the C terminal of the protein molecule, respectively: heavy chain signal peptide-heavy chain (HC) molecule of anti-HIV-gp160 broad-spectrum neutralizing antibody-Furion cleavage site-F2A self-splicing 2A sequence-light chain signal peptide-light chain (LC) molecule of anti-HIV-gp160 broad-spectrum neutralizing antibody. The heavy chain (HC) and light chain (LC) molecules of the sequence shown carry their own signal peptide sequences. The heavy chain and light chain of the antibody molecule are linked by the Furin splicing site and the self-splicing 2A sequence. The sequence shown was translated as a complete precursor molecule in cells as a whole. The self-splicing 2A sequence and the Furin splicing site were digested in the cytoplasm, respectively, resulting in highly accurate heavy chain and light chain molecules. When the heavy chain and light chain molecules were secreted from the cells, they were assembled into a monoclonal antibody with a complete structure and function in the cell supernatant. The structure of the expected mature molecule is shown in Fig. 2B, and the gene structure is shown in Fig. 3B.

### Example 2: Design of a gene expressing a neutralizing antibody against HIV

A total of four gene expression cassettes of anti-HIV neutralizing antibodies were co-constructed, respectively:
1. The anti-HIV broad-spectrum neutralizing antibody 10E8V4-5R+100cF (see SEQ ID No: 1 for the protein sequence) (see SEQ ID No: 2 for the DNA sequence)
2. The anti-HIV broad-spectrum neutralizing antibody PGT128 (see SEQ ID No: 3 for the protein sequence) (see SEQ ID No: 4 for the DNA sequence)
3. The bispecific anti-HIV neutralizing antibody-like molecule KL-BsHIV01 including the anti-HIV neutralizing antibody 10E8V4-5R+100cF-scfv (see SEQ ID No: 5 for the protein sequence) (see SEQ ID No: 6 for the DNA sequence)
4. The bispecific anti-HIV neutralizing antibody-like molecule KL-BsHIV02 including the anti-HIV neutralizing antibody PGT128-scfv (see SEQ ID No: 7 for the protein sequence) (see SEQ ID No: 8 for the DNA sequence)

1 and 2 include a heavy chain signal peptide, an anti-HIV-1-gp41-MPER (10E8V4-5R+100cF) heavy chain molecule, an anti-HIV-1-gp120-V3 loop (pgt128) heavy chain molecule; an Furion cleavage site; an F2A self-splicing 2A sequence; a light chain signal peptide; an anti-HIV-1-gp41-MPER (10E8V4-5R+100cF) light chain molecule, and an anti-HIV-1-gp120-V3 loop (pgt128) light chain molecule.

Respectively, the single chain variable region fragment (scfv) of the anti-HIV-gp160 broad-spectrum neutralizing antibody in 3 and 4 is: an anti-HIV-1-gp41-MPER (10E8V4-SR+100cF)-scfv, an anti-HIV-1-gp120-V3 loop (pgt128)-scfv; 3 and 4 each further includes a single chain variable region fragment scfv of the anti-human CD4 antibody (Ibalizumab), a human IgG1 CH2, and a human IgG1 CH3.

### Example 3: Gene therapy vector constructs of the HIV neutralizing antibodies

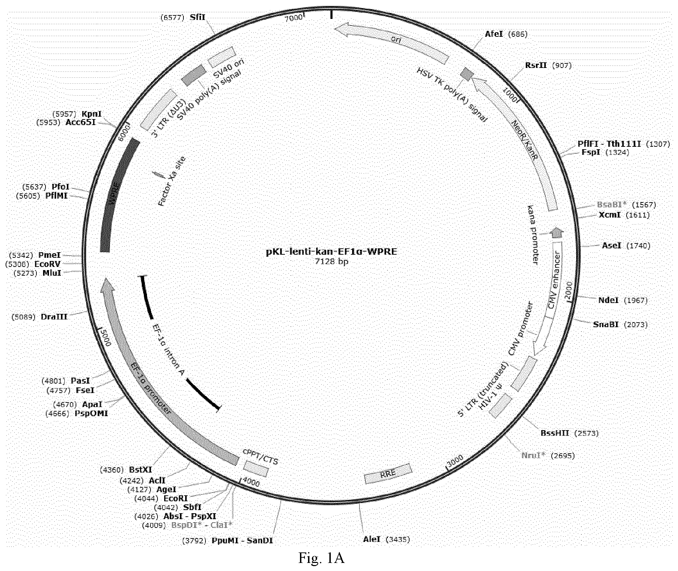

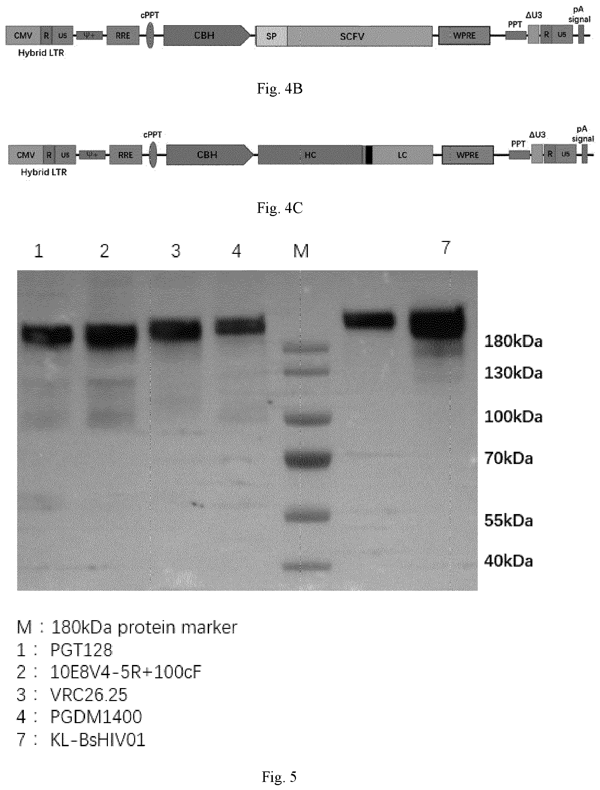

As shown in Figs. 4B and 4C, the above gene expression cassettes of the monoclonal antibody molecule were cloned into the latest generation of lentiviral vector pKL-kan-lenti-EF1α-WPRE (Fig. 1A) (see SEQ ID No: 9 for the DNA sequence) currently in use by multi-fragment recombination linking. The lentiviral vector includes: 5'LTR, in which the promoter region of the LTR is replaced by a CMV promoter; a Ψ packaging signal; a retrovirus output element RRE; cPPT; the promoter CBH; and a polynucleotide encoding a polypeptide of an antibody fragment of an HIV neutralizing antibody; and the post-transcriptional regulatory element wPRE; PPT; Δ U3 3'LTR; and a poly A signal. The gene expression cassettes of the neutralizing antibody designed in Example 2 were synthesized by GenScript Biotech Corporation and then cloned between the multi-cloning sites EcoRI/EcoRV on the lentiviral vector framework pKL-kan-lenti-EF1α-WPRE by homologous recombination. After cloning, the sequence information was confirmed by sequencing, and the plasmids were named respectively as: pKL-Kan-lenti-CBH-KL-BsHIV01 (SEQ ID No: 11); pKL-Kan-lenti-CBH-10E8V4-SR+100cF (SEQ ID No: 12); pKL-Kan-lenti-CBH-KL-BsHIV02 (SEQ ID No: 13); and pKL-Kan-lenti-CBH-PGT128 (SEQ ID No: 14). The sequence of CBH promoter is SEQ ID No: 10.

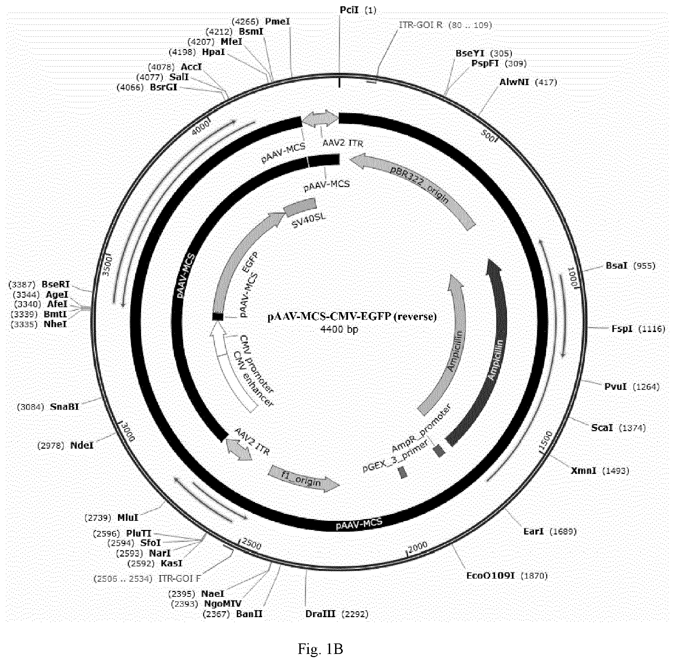

As shown in Fig. 4A, BsHIV01 and BsHIV02, the gene expression cassettes of the HIV neutralizing antibodies, existing in pKL-Kan-lenti-CBH-KL-BsHIV01, were cloned between the multi-cloning sites Mlul/Sall of the latest generation of adeno-associated viral vector pAAV-MCS-CMV-EGFP (reverse) (SEQ ID No: 15) (see Fig. 1B) currently in use by mono-fragment recombination linking. The adeno-associated viral vector included AAV2 ITR, the promoter CBH; a polynucleotide encoding an HIV neutralizing antibody fragment; the SV40 polyA signal, and AAV2 ITR. The plasmids were named as pAAV-CBH-KL-BsHIV01 (SEQ ID No: 16) and pAAV-CBH-KL-BsHIV02 (SEQ ID No: 17).

### Example 4: Packaging of the viral vector constructs and verification of cell line expression

The lentivirus expression vectors (pKL-Kan-lenti-CBH-KL-BsHIV01, pKL-Kan-lenti-CBH-10E8V4-SR+100cF, pKL-Kan-lenti-CBH-KL-BsHIV02 and pKL-Kan-lenti-CBH-PGT128) were used to carry out the packaging of gene therapy lentiviral vectors of antibodies in 293T cell lines. The gene expression lentiviral vector of the monoclonal antibody molecule constructed in Example 3, an envelope plasmid (pKL-Kan-Vsvg, the nucleotide sequence of which is shown as SEQ ID NO: 18) and a packaging plasmid (pKL-Kan-Rev, the nucleotide sequence of which is shown as SEQ ID NO: 19; and pKL-Kan-GagPol, the nucleotide sequence of which is shown as SEQ ID NO: 20) were mixed, and then were used to co-transfect 293T cells (purchased from American Type Culture Collection (ATCC), with the ATCC deposit number of CRL-3216) at the same time, and the packaging of the gene therapy lentivirus of the HIV neutralizing antibody was carried out in the 293T cell lines. The transfection method was instantaneous transfection of eukaryotic cells mediated by a PEI cationic polymer. The PEI cationic polymer was a PEI-Max transfection reagent (from Polysciences, product number: 24765-1). The transfection operation was carried out according to the standard operation recommended by the manufacturer, and the transfection scale was 2×15 cm² cell culture dishes. 48 hours after transfection, the lentiviral vector (the transfected cell culture supernatant) was harvested. First, the cell debris was removed by centrifugation at room temperature and 4000 rpm for 5 minutes in a desktop bucket centrifuge, and then the virus particle precipitate was obtained by centrifugation at 4 °C and 10000 g for 4 hours. After the supernatant was removed, 1 ml of the DMEM complete medium was added into the virus particle precipitate, the virus particles were resuspended by a microsyringe, and the prepared virus resuspension was sub-packaged and cryopreserved at -80 °C for later use.

Then the packaged virus was used to infect the 293T cells. The culture supernatant of cells infected with the lentivirus was loaded into SDS-PAGE, and the goat anti-human IgG Fc (KPL, product number: 04-10-20) labeled with HRP was used as the primary antibody. The results of Westernblotting (Fig. 5) show that different lentiviral vectors of the HIV neutralizing antibody can all effectively express the HIV neutralizing antibody after in vitro transduction of cells, and secrete the mature antibody protein into the cell culture supernatant.

The AAV expression vectors (pAAV-CBH-KL-BsHIV01 and pAAV-CBH-KL-BsHIV02) were used to carry out the packaging of gene therapy AAV vectors of antibodies in 293T cell lines. The antibody gene AAV vectors (pAAV-CBH-KL-BsHIV01 and pAAV-CBH-KL-BsHIV02) constructed in Example 2, an envelope plasmid (AAV2/8, the nucleotide sequence of which is shown as SEQ ID NO: 21) and a packaging plasmid (pHelper, the nucleotide sequence of which is shown as SEQ ID NO: 22) were mixed, and then were used to co-transfect 293T cells at the same time, and the packaging of the gene therapy vector AAV of the HIV neutralizing antibody was carried out in the 293T cell lines. The transfection method was instantaneous transfection of eukaryotic cells mediated by a PEI cationic polymer. The PEI cationic polymer was a PEI-Max transfection reagent (from Polysciences, product number: 24765-1). The transfection operation was carried out according to the standard operation recommended by the manufacturer, and the transfection scale was 15 cm cell culture dishes. 7 hours after transfection, the supernatant was pipetted out, and replaced with 25 ml of a toxin-producing medium. 120 hours after transfection, the supernatant and cells were collected, and centrifuged at 4200 rpm for 10 min. After centrifugation, the supernatant and cells were separated. A lysis solution and a ribozyme were added into the cells, which were lysed and digested for 1 h, and centrifuged at 10000 g for 10 min to obtain the supernatant of the lysate. The supernatant of the lysate and the supernatant of the culture medium were purified by affinity chromatography, and then sub-packaged and cryopreserved at -80°C for later use.

### Example 5: Function verification of the HIV neutralizing antibody expressed in the supernatant of the cells transfected by the gene therapy vectors

After HIV infects TZMbl cells, the expression of the luciferase protein in the TZMbl cells is significantly stimulated. If an HIV neutralizing antibody neutralizes the HIV virus in the supernatant, the TZMbl cells will not express the luciferase protein. The neutralization effect of an HIV neutralizing antibody is determined by measuring RLU (relative light unit) in the cell lysate with the firefly luciferase reporter gene detection kit (Beyotime Biotechnology, product number: RC006). A higher RLU shows a worse neutralization effect of the antibody; and a lower RLU shows a better neutralization effect of the antibody.

The packaged lentiviruses (pKL-Kan-lenti-CBH-KL-BsHIV01, pKL-Kan-lenti-CBH-10E8V4-5R+100cF, pKL-Kan-lenti-CBH-KL-BsHIV02 and pKL-Kan-lenti-CBH-PGT128) were used to infect 293T cells with different MOI. The supernatant and part were collected at 48 hours, and the expression of the HIV neutralizing antibody in the supernatant was detected by ELISA. The copy number of lentivirus infection was detected by the probe method.

1. The 293T cells infected by the lentivirus were collected. After washing with PBS, the cells were collected by centrifuging at 4200 rpm for 5min, resuspended in 20 ul of a rapid extraction solution (QE DNA Extraction Solution), and the following procedures were run with a PCR instrument to lyse the cells and extract the total DNA.

**Table 1 PCR procedure**

| Temperature | Time |
|---|---|
| 65°C | 15 min |
| 68°C | 15 min |
| 95°C | 10 min |

The Vector Copy Number (VCN) of the 293T cells infected by the lentivirus was calculated by quantitative PCR with methods well known in the art.

The primer probe sequences used in quantitative PCR were as follows:

V Forward primer 5'-AGTAAGACCACCGCACAGCA-3' (SEQ ID NO.23)

LV Reverse primer 5'-CCTTGGTGGGTGCTACTCCT-3' (SEQ ID NO.24)

LV probe 5'-CCTCCAGGTCTGAAGATCAGCGGCCGC-3' (SEQ ID NO.25)

HK Forward primer 5'-GCTGTCATCTCTTGTGGGCTGT-3' (SEQ ID NO.26)

HK probe 5'-CCTGTCATGCCCACACAAATCTCTCC-3' (SEQ ID NO.27)

HK Reverse primer 5'-ACTCATGGGAGCTGCTGGTTC-3' (SEQ ID NO.28)

The LV probe has a 6FAM fluorescent group in its 5' terminal and a TAMRA fluorescent group in its 3' terminal.

The HK probe has a CY5 fluorescent group in its 5' terminal and a DGB fluorescent group in its 3' terminal.

The running procedure of quantitative PCR was: 94°C 5 min; 95°C 10 s, 60°C 30 s, 40 cycles. The results of VCN are shown in Table 3.

Table 3 Neutralization of the virus by the cell culture supernatant transduced by the gene therapy vector of the HIV neutralizing antibody

| | VCN | AD-8 RLU | pNL4-3 RLU |
|---|---|---|---|
| PGT128 | 0.99 | 1.47E+05 | 4.74E+05 |
| 10E8V4-5R+100cF | 0.64 | 9.76E+05 | 1.21E+05 |
| KL-BsHIV02 | 1.14 | 1.29E+04 | 2.10E+05 |
| KL-BsHIV01 | 0.57 | 8.54E+03 | 9.93E+03 |
| PC | | 6.45E+05 | 4.07E+05 |
| NC | | 7.39E+03 | |

2. The Tzmbl cells were plated at 2E4cells/well. 50 uL of the antibody expression supernatants of different HIV neutralizing antibodies with the same VCN were each mixed with 50 uL of the HIV virus pAD-8 or pNL4-3, incubated at 37°C for 30 min, and added into tzmbl cells. The wells added only pAD-8 or pNL4-3 were used as positive controls, and the wells without HIV were used as negative controls. At 24 h, the supernatant was discarded, and 100 uL of the cell lysate was added. After 10 min, the lysed cells were collected and centrifuged at 8000 rpm for 5 min. 100 uL of the firefly luciferase detection reagent was added into 50 uL of the lysed cells. RLU (relative light unit) was determined by a multifunctional microplate reader with chemiluminescence function. The results of in vitro cell trial show (see Table 3) that in the culture supernatant of 293T cells transduced by the gene therapy vector of the HIV neutralizing antibody BsHIV01 there are antibodies that completely neutralize HIV, and the neutralization effect is significantly better than that of the gene therapy vector of the HIV neutralizing antibody 10E8V4-SR+100cF.

### Example 6: Function verification of the HIV neutralizing antibody expressed in the supernatant of the cells transfected by the gene therapy vectors

The packaged lentivirus vetors pKL-Kan-lenti-CBH-KL-BsHIV01 and pKL-Kan-lenti-CBH-10E8V4-SR+100cF were used to infect 293T cells with different MOI. The supernatant and part were collected at 48 hours, and the expression of the HIV neutralizing antibody in the supernatant was detected by ELISA. The copy number of lentiviral vector infection was detected by the probe method.
1. The 293T cells infected by the lentiviral vector were collected. After washing with PBS, the cells were collected by centrifuging at 4200 rpm for 5min, resuspended in 20 ul of a rapid extraction solution (QE DNA Extraction Solution), and the following procedures were run with a PCR instrument to lyse the cells and extract the total DNA.

**Table 2 PCR procedure**

| Temperature | Time |
|---|---|
| 65°C | 15min |
| 68°C | 15min |
| 95°C | 10 min |

The Vector Copy Number of the 293T cells infected by the lentivirus was calculated by quantitative PCR with methods well known in the art. Reference is made to Example 5.

The results of VCN are shown in Table 4.

**Table 4 ELISA detection of the expression level of the cell culture supernatant transduced by the gene therapy vector of the HIV neutralizing antibody**

| VCN | | Expression concentration (ng/mL) | |
|---|---|---|---|
| 10E8V4-5R+100cF | KL-BsHIV01 | 10E8V4-5R+100cF | KL-BsHIV01 |
| 0.14 | 0.04 | <Min | <Min |
| 0.09 | 0.05 | 81.18 | <Min |
| 0.16 | 0.07 | 88.50 | <Min |
| 0.20 | 0.09 | 117.70 | 65.34 |
| 0.25 | 0.13 | 171.82 | 116.66 |
| 0.31 | 0.18 | 216.37 | 134.70 |
| 0.47 | 0.20 | 316.36 | 154.61 |
| 0.48 | 0.20 | 328.57 | 198.28 |
| 0.60 | 0.38 | 597.85 | 294.47 |
| 0.95 | 0.40 | 1188.55 | 336.60 |
| 1.20 | 0.66 | 1261.70 | 465.47 |
| 1.39 | 0.64 | 1480.60 | 586.85 |

2. The microplate (corning, product number: 42592) was coated with the synthetic HIV MPER polypeptide, with the KL-BsHIV01 standard for the expression supernatant and purification and quantification as the primary antibody, and the goat anti-human IgGFc labeled with HRP (KPL, product number: 04-10-20) as the secondary antibody. The color was developed by TMB, and the OD value at 450 nm was detected by a microplate reader. The results of ELISA (Table 4) show that lentiviral vectors of the anti-HIV neutralizing antibody can effectively express the HIV neutralizing antibody after in vitro transduction of cells, and secrete the mature HIV neutralizing antibody protein into the cell culture supernatant. When the VCNs are the same, there is little difference in the expression levels of BsHIV01 and 10E8V4-SR+100cF in the supernatant.

3. The Tzmbl cells were plated at 2E4cells/well. 50 uL of the antibody expression supernatants were mixed with 50 uL of the HIV pAD-8 or pNL4-3, incubated at 37°C for 30 min, and added into tzmbl cells. The wells added only pAD-8 or pNL4-3 were used as positive controls, and the wells without HIV were used as negative controls. At 24 h, the supernatant was discarded, and 100 uL of the cell lysate was added. After 10 min, the lysed cells were collected and centrifuged at 8000 rpm for 5 min. 100 uL of the firefly luciferase detection reagent was added into 50 uL of the lysed cells. RLU (relative light unit) was determined by a multifunctional microplate reader with chemiluminescence function. The results of in vitro cell trial show (see Table 5) that, in the culture supernatant of 293T cells transduced by the gene therapy vector of the HIV neutralizing antibody BsHIV01 there are antibodies that completely neutralize HIV, and the neutralization effect is significantly better than that of the gene therapy vector of the HIV neutralizing antibody 10E8V4-SR+100cF.

**Table 5 The Neutralization effect of the virus by the cell culture supernatant transduced by the gene therapy vector of the HIV neutralizing antibody**

| pNL4-3 | | AD-8 | |
|---|---|---|---|
| 10E8V4-5R+100cF | KL-BsHIV01 | 10E8V4-5R+100cF | KL-BsHIV01 |
| 9.28E+04 | 9.52E+03 | 1.42E+06 | 1.54E+05 |
| 8.18E+04 | 7.97E+03 | 1.63E+06 | 8.59E+04 |
| 9.00E+04 | 7.50E+03 | 1.48E+06 | 4.47E+04 |
| 7.87E+04 | 9.89E+03 | 1.81E+06 | 3.63E+04 |
| 6.68E+04 | 9.79E+03 | 1.58E+06 | 1.90E+04 |
| 5.79E+04 | 9.62E+03 | 7.88E+05 | 1.20E+04 |
| 4.20E+04 | 9.02E+03 | 1.00E+06 | 1.25E+04 |
| 3.55E+04 | 9.42E+03 | 1.45E+06 | 8.16E+03 |
| 3.49E+04 | 9.80E+03 | 1.20E+06 | 7.48E+03 |
| 2.15E+04 | 1.01E+04 | 7.79E+05 | 6.56E+03 |
| 2.26E+04 | 9.46E+03 | 7.30E+05 | 6.20E+03 |
| 1.98E+04 | 8.81E+03 | 8.56E+05 | 7.27E+03 |
| PC | 9.39E+04 | PC | 7.27E+05 |
| NC | 7.29E+03 | | |

### Example 7: Intramuscular injection of balbc mice with an adeno-associated virus gene therapy vector

An AAV gene therapy vector (pAAV-CBH-KL-BsHIV01) was injected into the thigh muscles of the hind limbs of mice by intramuscular injection at the dosage of 1E12vg/mouse and 5E12vg/mouse. Blood was taken every a few weeks, serum was separated, and the antibody concentration expressed in serum was detected by the ELISA kit according to the instructions.

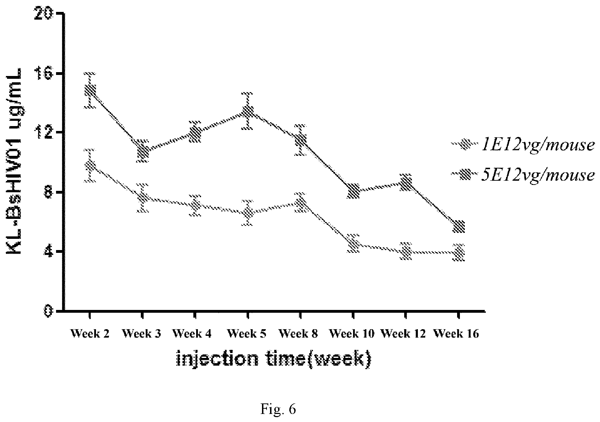

The results of ELISA (Fig. 6) show that the adeno-associated viral vector of the anti-HIV neutralizing antibody KL-BsHIV01 can continuously and effectively express the HIV neutralizing antibody in mice.

The above examples are intended to illustrate the disclosed embodiments of the present disclosure, and should not be construed as limiting the present disclosure. In addition, various modifications listed herein and changes in methods in the present disclosure are obvious to those skilled in the art without departing from the scope and spirit of the present disclosure. Although the present disclosure has been described in detail with reference to various specific preferred examples, it should be understood that the present disclosure should not be limited to these specific examples. In fact, various modifications as mentioned above that are obvious to those skilled in the art to obtain the present disclosure should be included in the scope of the present disclosure.

## Claims

1. A nucleic acid construct, wherein the nucleic acid construct comprises a plurality of polynucleotides encoding single chain variable region fragments of an anti-AIDS neutralizing antibody, and a polynucleotide encoding an immunoglobulin Fc fragment; the single chain variable region fragments of the anti-AIDS neutralizing antibody comprise a single chain variable region fragment capable of specifically binding to HIV, and a single chain variable region fragment capable of specifically binding to a CD4 receptor.

2. The nucleic acid construct according to claim 1, wherein the single chain variable region fragment capable of specifically binding to HIV is a single chain variable region fragment of antibody capable of specifically binding to the following region: the V1V2 glycan region, the V3 glycan region, gp160, gp120, the junction site of gp120 and gp41, or the membrane proximal external region on gp41; preferably, the single chain variable region fragment of antibody capable of specifically binding to the V3 glycan region is PGT128; preferably, the single chain variable region fragment of antibody capable of specifically binding to the membrane proximal external region on gp41 is a single chain variable region fragment of 10E8V4-5R+100cF.

3. The nucleic acid construct according to claim 1, wherein the single chain variable region fragment capable of specifically binding to the CD4 receptor is selected from single chain variable region fragments of ibalizumab.

4. The nucleic acid construct according to claim 1, wherein the nucleic acid construct is a non-viral vector or a viral vector, and the viral vector is preferably a lentiviral vector or an adeno-associated viral vector, and each polynucleotide is located in an expression cassette of the lentiviral vector or the adeno-associated viral vector.

5. The nucleic acid construct according to claim 4, wherein the nucleotide sequence of the nucleic acid construct is shown as SEQ ID NO: 6 or SEQ ID NO: 8.

6. A lentivirus, wherein the lentivirus is formed by packaging the nucleic acid construct according to any one of claims 1 to 5 by a virus.

7. A lentiviral vector system, wherein the lentiviral vector system comprises the nucleic acid construct according to any one of claims 1 to 5 and an auxiliary plasmid.

8. The lentiviral vector system according to claim 7, wherein the lentiviral vector system further comprises a host cell.

9. An adeno-associated virus, wherein the adeno-associated virus is formed by packaging the nucleic acid construct according to any one of claims 1 to 5 by a virus.

10. An adeno-associated viral vector system, wherein the adeno-associated viral vector system comprises the nucleic acid construct according to any one of claims 1 to 5 and an auxiliary plasmid.

11. The adeno-associated viral vector system according to claim 10, wherein the adeno-associated viral vector system further comprises a host cell.

12. A bispecific antibody for preventing or treating AIDS, wherein the bispecific antibody is an antibody produced by infecting host cells with the lentivirus according to claim 6 or the adeno-associated virus according to claim 9; preferably, the amino acid sequence of the bispecific antibody is shown as SEQ ID NO: 5 or SEQ ID NO: 7.

13. A composition for preventing or treating AIDS, wherein the effective substances of the composition comprise the nucleic acid construct according to any one of claims 1 to 5; and/or the lentivirus according to claim 6, and/or the adeno-associated virus according to claim 9.

14. A cell line, wherein the cell line is infected by the lentivirus according to claim 6 or the adeno-associated virus according to claim 9.

15. Use of the nucleic acid construct according to any one of claims 1 to 5, the lentivirus according to claim 6, the lentiviral vector system according to claim 7 or 8, the adeno-associated virus according to claim 9, the antibody according to claim 12 or the cell line according to claim 14 in preparing drugs for preventing and/or treating AIDS.

16. A method for treating AIDS, wherein the method comprises administering an effective amount of the bispecific antibody according to claim 12 to a patient.
